# EUROPEAN PATENT SPECIFICATION

(11) **EP 3 600 223 B1**
(45) Date of publication and mention of the grant of the patent: **07.06.2023**
(21) Application number: 17828846.0
(22) Date of filing: 07.12.2017
(51) Int. Cl.: A61K 8/02, A61K 8/25, A61K 8/34, A61K 8/60, A61Q 19/10

(54) **ANHYDROUS EXFOLIATING COMPOSITION COMPRISING C3-C10 DIOLS**
WASSERFREIE EXFOLIATIONSZUSAMMENSETZUNG MIT C3-C10-DIOLEN
COMPOSITION EXFOLIANTE ANHYDRE COMPRENANT DES C3-C10 DIOLS

(30) Priority: 09.12.2016 FR 1662216
(43) Date of publication of application: 05.02.2020
(73) Proprietor: L'OREAL, 75008 Paris (FR)
(72) Inventor: PERRAULT, Véronique, 94152 Chevilly La Rue (FR); NOEL-POQUET, Christine, 94152 Chevilly La Rue (FR)
(74) Representative: Cabinet Nony
(86) International application number: PCT/EP2017/081785
(87) International publication number: WO 2018/104428

(56) References cited:
- WO-A1-2005/063198
- WO-A1-2013/190129

## Description

The present invention relates to the field of exfoliating compositions for the body and/or the face.

Exfoliation is a care of the skin which consists in removing, by a mechanical rubbing action, the dead cells of the surface layers of the epidermis in order to improve the texture and the flesh tone thereof, in particular to render the skin smoother, to accelerate cell replacement of the skin and to improve the penetration of cosmetic active principles.

Reference is also made to scrubbing the skin.

Exfoliating compositions can be aqueous or anhydrous; in addition, the exfoliating particles used can be of very diverse natures.

One of the objectives of the present invention is to provide an oily composition comprising exfoliating particles which exhibits a viscosity appropriate for being packaged in ajar and making possible good grasping with the finger, and also easy use, in particular easy spreading.

Said composition should in addition exhibit satisfactory properties of homogeneity; in particular, the composition should not exhibit phase separation or deposition of the exfoliating particles at the bottom of the container.

The composition should also be satisfactory visually and exhibit effective antimicrobial protection.

This is because, insofar as said exfoliating compositions are intended to be used in humid surroundings, such as bathrooms or spas, they should exhibit a good bactericidal cover in order to prevent microbial contaminations. In point of fact, the preservatives provided for this purpose are generally hydrophilic and consequently difficult to formulate in an oily medium; in addition, diols, in particular propane-1,3-diol and pentane-1,2-diol, are preservatives known for their instability.

Patent application WO2005/063198 already describes an anhydrous exfoliating composition for the skin comprising an emollient oil, an ionic surfactant, an exfoliating agent and a gelling agent for the composition; however, this patent application does not mention the use of the diols used in the composition according to the invention.

The composition according to the present invention exhibits the advantage of using diols as preservatives in a stable anhydrous composition comprising exfoliating particles.

Thus, the present text describes an anhydrous, in particular cosmetic, composition comprising, in an oily fatty phase, at least:
a) a C₃-C₁₀ diol,
b) hydrophobic silica aerogel particles,
c) exfoliating particles,
said composition exhibiting, at a temperature of 25°C, a viscosity of between 100 and 200 poises (respectively 10 Pa.s and 20 Pa.s).

In particular, the present invention is targeted at an anhydrous, in particular cosmetic, composition comprising, in an oily fatty phase, at least:
a) a C₃-C₁₀ diol,
b) hydrophobic silica aerogel particles in a solids content ranging from 3.7% to 6% by weight with respect to the total weight of the composition,
c) exfoliating particles in an amount ranging from 10% to 50% by weight with respect to the total weight of the composition,
said composition exhibiting, at a temperature of 25°C, a viscosity of between 10 and 20 Pa.s.

This composition is stable and homogenous; in particular, no phase separation is observed, that is to say no appearance of an oily layer at the surface or deposition of exfoliating particles at the bottom of the container is observed after storage of the composition according to the stability criteria of an oily exfoliant, in particular at 45°C for three weeks.

The composition according to the invention exhibits good properties, both cosmetically and visually, and also good antimicrobial protection.

The composition obtained is a supple and homogenous paste, with a setting appropriate to its use, a pleasant appearance and a pleasant application.

On use, the product leaves a soft and smooth skin.

According to another aspect, the present invention is targeted at a method for the cosmetic treatment of the skin of the body and/or of the face, comprising a stage of application of a cosmetic composition as defined above to the skin, preferably followed by a stage of rinsing off said composition, preferentially with water.

The term "anhydrous" is understood to mean, within the meaning of the present invention, that the composition comprises less than 2% by weight of water; preferably, it is devoid of water.

### Viscosity

The texture of the composition can be characterized by viscosity measurements.

### Protocol for measuring the viscosity

The viscosity measurement is generally carried out at 25°C, using a Rheomat RM180 viscometer equipped with a No. 4 spindle, the measurement being carried out after 10 minutes of rotation of the spindle in the composition (after which time stabilization of the viscosity and of the speed of rotation of the spindle are observed), at a shear rate of 200 s⁻¹.

The composition according to the invention advantageously exhibits a thick texture on "setting"; more particularly, it has a viscosity ranging from 100 to 200 poises, preferably ranging from 125 to 200 poises and preferentially ranging from 150 to 200 poises.

### Diols

The composition according to the invention comprises at least one C₃-C₁₀ diol.

The diol can be chosen from propane-1,3-diol, pentane-1,2-diol, octane-1,2-diol, decane-1,2-diol and their mixtures.

Preferably, the diol is chosen from propane-1,3-diol, pentane-1,2-diol and their mixtures.

Preferably, the composition comprises a C₃-C₁₀ diol in a content ranging from 1% to 10% by weight, preferably ranging from 2% to 8% by weight and preferentially ranging from 3% to 7% by weight, with respect to the total weight of the composition.

### Hydrophobic silica aerogels

The composition according to the invention also comprises silica aerogel particles as gelling agent.

Silica aerogels are porous materials obtained by replacing (by drying) the liquid component of a silica gel with air.

They are generally synthesized via a sol-gel process in a liquid medium and then dried, usually by extraction with a supercritical fluid, the one most commonly used being supercritical CO₂. Drying of this type makes it possible to avoid contraction of the pores and of the material. The sol-gel process and the various drying operations are described in detail in Brinker C.J. and Scherer G.W., Sol-Gel Science, New York, Academic Press, 1990.

The hydrophobic silica aerogel particles used in the present invention exhibit a specific surface per unit of weight (Sw) ranging from 500 to 1500 m²/g, preferably from 600 to 1200 m²/g and better still from 600 to 800 m²/g, and a size, expressed as volume-average diameter (D[0.5]), ranging from 1 to 1500 µm, better still from 1 to 1000 µm, preferably from 1 to 100 µm, in particular from 1 to 30 µm, more preferably from 5 to 25 µm, better still from 5 to 20 µm and even better still from 5 to 15 µm.

According to one embodiment, the hydrophobic silica aerogel particles used in the present invention exhibit a size, expressed as volume-average diameter (D[0.5]), ranging from 1 to 30 µm, preferably from 5 to 25 µm, better still from 5 to 20 µm and even better still from 5 to 15 µm.

The specific surface per unit of weight can be determined by the nitrogen adsorption method, known as the BET (Brunauer-Emmett-Teller) method, described in The Journal of the American Chemical Society, vol. 60, page 309, February 1938, and corresponding to international standard ISO 5794/1 (Annex D). The BET specific surface corresponds to the total specific surface of the particles under consideration.

The sizes of the silica aerogel particles can be measured by static light scattering using a commercial particle size analyzer of MasterSizer 2000 type from Malvern. The data are processed on the basis of the Mie scattering theory. This theory, which is exact for isotropic particles, makes it possible to determine, in the case of non-spherical particles, an "effective" particle diameter. This theory is described in particular in the publication by Van de Hulst, H.C., Light Scattering by Small Particles, Chapters 9 and 10, Wiley, New York, 1957.

According to an advantageous embodiment, the hydrophobic silica aerogel particles used in the present invention exhibit a specific surface per unit of weight (Sw) ranging from 600 to 800 m²/g.

The silica aerogel particles used in the present invention can advantageously exhibit a packed density ρ ranging from 0.02 g/cm³ to 0.10 g/cm³, preferably from 0.03 g/cm³ to 0.08 g/cm³ and in particular ranging from 0.05 g/cm³ to 0.08 g/cm³.

In the context of the present invention, this density can be assessed according to the following protocol, known as the packed density protocol:
40 g of powder are poured into a graduated measuring cylinder and then the measuring cylinder is placed on a Stav 2003 device from Stampf Volumeter; the measuring cylinder is subsequently subjected to a series of 2500 packing actions (this operation is repeated until the difference in volume between two consecutive tests is less than 2%) and then the final volume Vf of packed powder is measured directly on the measuring cylinder. The packed density is determined by the ratio w/Vf, in this case 40/Vf (Vf being expressed in cm³ and w in g).

According to a preferred embodiment, the hydrophobic silica aerogel particles used in the present invention exhibit a specific surface per unit of volume Sv ranging from 5 to 60 m²/cm³, preferably from 10 to 50 m²/cm³ and better still from 15 to 40 m²/cm³.

The specific surface per unit of volume is given by the relationship: Sv = Sw x ρ; where ρ is the packed density, expressed in g/cm³, and Sw is the specific surface per unit of weight, expressed in m²/g, as defined above.

Preferably, the hydrophobic silica aerogel particles used according to the invention have an oil absorption capacity, measured at the wet point, ranging from 5 to 18 ml/g, preferably from 6 to 15 ml/g and better still from 8 to 12 ml/g.

The absorption capacity, measured at the wet point and denoted Wp, corresponds to the amount of oil which it is necessary to add to 100 g of particles in order to obtain a homogeneous paste.

It is measured according to the "wet point" method or the method for determining the oil uptake of a powder described in standard NF T 30-022. It corresponds to the amount of oil adsorbed onto the available surface of the powder and/or absorbed by the powder by measurement of the wet point, described below:
An amount w = 2 g of powder is placed on a glass plate and then the oil (isononyl isononanoate) is added dropwise. After addition of 4 to 5 drops of oil to the powder, mixing is carried out using a spatula, and addition of oil is continued until conglomerates of oil and powder have formed. From this point, the oil is added at the rate of one drop at a time and the mixture is subsequently triturated with the spatula. The addition of oil is stopped when a firm and smooth paste is obtained. This paste must be able to be spread over the glass plate without cracks or the formation of lumps. The volume Vs (expressed in ml) of oil used is then noted.

The oil uptake corresponds to the ratio Vs/w.

The aerogels used according to the present invention are aerogels of hydrophobic silica, preferably of silylated silica (INCI name: silica silylate).

The term *"hydrophobic silica"* is understood to mean any silica whose surface is treated with silylating agents, for example halogenated silanes, such as alkylchlorosilanes; siloxanes, in particular dimethylsiloxanes, such as hexamethyldisiloxane; or silazanes, so as to functionalize the OH groups with silyl groups Si-Rn, for example trimethylsilyl groups.

As regards the preparation of hydrophobic silica aerogel particles surface-modified by silylation, reference may be made to the document US 7 470 725.

Use will preferably be made of hydrophobic silica aerogel particles surface-modified with trimethylsilyl groups, preferably with the INCI name Silica silylate.

Mention may made, as hydrophobic silica aerogels which can be used in the invention, for example, of the aerogel sold under the name VM-2260 or VM-2270 (INCI name: Silica silylate) by Dow Corning, the particles of which exhibit a mean size of approximately 1000 microns and a specific surface per unit of weight ranging from 600 to 800 m²/g.

Mention may also be made of the aerogels sold by Cabot under the references Aerogel TLD 201, Aerogel OGD 201, Aerogel TLD 203, Enova^{®} Aerogel MT 1100 and Enova Aerogel MT 1200.

Use will preferably be made of the aerogel sold under the name VM-2270 (INCI name: Silica silylate) by Dow Corning, the particles of which exhibit a mean size ranging from 5-15 microns and a specific surface per unit of weight ranging from 600 to 800 m²/g.

The hydrophobic silica aerogel particles are present in the composition according to the invention in a solids content ranging from 3.7% to 6% by weight, preferably from 4% to 5.5% by weight and preferably from 4% to 5% by weight, with respect to the total weight of the composition.

The composition according to the present invention can also comprise at least one other additional lipophilic thickener other than the hydrophobic silica aerogel particles, in particular chosen from esters of dextrin and of a fatty acid, preferably a C₁₂ to C₂₄ and in particular C₁₄-C₁₈ fatty acid, or their mixtures and (poly)esters of at least one fatty acid comprising from 20 to 24 carbon atoms and of glycerol.

Preferably, the dextrin ester is an ester of dextrin and of a C₁₂-C₁₈ and in particular C₁₄-C₁₈ fatty acid.

Preferably, the dextrin ester is chosen from dextrin myristate and/or dextrin palmitate, and their mixtures, and more preferably dextrin palmitate.

According to a specific embodiment, the dextrin ester is dextrin myristate, such as that sold in particular under the name of Rheopearl MKL-2 by Chiba Flour Milling.

According to a preferred embodiment, the dextrin ester is dextrin palmitate. The latter can, for example, be chosen from those sold under the names Rheopearl^{®} TL 2 OR, Rheopearl^{®} KL 2 or Rheopearl^{®} KE by Chiba Flour Milling.

The composition according to the invention can comprise between 0.1% and 2% by total weight of dextrin ester(s) and preferably between 0.1% and 1% by weight, with respect to the total weight of the composition.

According to the invention, the (poly)ester of fatty acid and of glycerol is advantageously a (poly)ester of behenic acid and of glycerol, in particular a mono-, di- or triester, indeed even a mixture of these.

The (poly)ester of fatty acid and of glycerol is preferably a mixture of mono-, di- and/or triesters of behenic acid and of glycerol.

The ester of behenic acid and of glycerol is chosen in particular from the group constituted of glyceryl monobehenate, glyceryl dibehenate and glyceryl tribehenate, in particular a mixture of glyceryl monobehenate, glyceryl dibehenate and glyceryl tribehenate.

Such a mixture is in particular available, for example, under the INCI name "glyceryl dibehenate *&* tribehenin *&* glyceryl behenate" and is in particular sold under the reference Compritol 888 by Gattefossé.

The composition according to the invention can comprise between 0.2% and 4% by total weight of (poly)ester of behenic acid and of glycerol and preferably between 0.5% and 2% by weight, with respect to the total weight of the composition.

### Exfoliating agent

The composition according to the invention comprises exfoliating particles capable of resulting in a scrubbing of the skin.

Use may be made, as exfoliating particles, of exfoliating or scrubbing particles of mineral, vegetable or organic origins. They can also be of natural or synthetic origin.

Use may be made of particles of sugars, such as particles of saccharose, of sucrose, of fructose, of glucose, of galactose or of maltose and preferably of saccharose, of sucrose.

Thus, use may be made, for example, of perlite beads or powder, such as those sold under the name Imercare 270P-Scrub or Imercare 400P-Scrub or Imercare 800P-Scrub by Imerys.

It is also possible to use, for example, polyethylene beads or powder, such as those sold under the name Microthene 35 Mesh MN 711-20 or Microthene MN 710-20 by Equistar or Cerapure 106C Coathylene NB 6081 from Shamrock Technologies Dupont or Flobeads CL5007 from Sumitomo Seika; polyamide (Nylon^{®}) particles, such as those sold by Arkema under the name Orgasol 2002 Exd Nat Cos; fibers, such as polyamide fibers, such as those sold by Utexbel under the name Pulpe Polyamide 12185 Taille 0.3 mm; polyvinyl chloride powder; pumice stone (INCI name: pumice), such as pumice 3/B from Eyraud; ground shells of fruit kernels, such as ground apricot kernels or walnut shells; sawdust; sand; loofah powder; glass beads; alumina (aluminum oxide) (INCI name: Alumina), such as the product sold under the name Dermagrain 900 by Marketech International; and their mixtures.

The composition according to the invention can also contain, as exfoliating agent, salts of NaCl type, more particularly sea salts, such as Dead Sea salt or Camargue salt.

Use is preferably made of particles of sugars, in particular of saccharose, of sucrose.

These exfoliating particles can be present in an amount ranging, for example, from 1% to 50% by weight, preferably from 10% to 50% by weight and better still from 25% to 45% by weight, with respect to the total weight of the composition.

### Nonionic surfactant

The composition of the invention comprises at least one nonionic surfactant chosen from polyoxyethylenated diesters or triesters of fatty acids and of glycerol having from 2 to 60 ethylene oxide units.

The nonionic surfactant facilitates the rinsing off with water of the composition after its application to the skin.

The fatty acid preferably comprises from 12 to 20 carbon atoms, preferably 16 to 20 carbon atoms and preferentially 18 carbon atoms. The fatty acid is preferably chosen from stearic acid, isostearic acid, oleic acid or myristic acid and is preferably stearic acid.

Mention may be made, as oxyethylenated esters of fatty acid and of glycerol, for example, of PEG-20 glyceryl triisostearate (INCI name: PEG-20 glyceryl triisostearate), such as that sold by Nihon Emulsion under the names Emalex GWIS-305 and Emalex GWIS-320EX.

Mention may also be made of nonionic surfactants chosen from the following products (INCI name/commercial name/supplier):
- PEG-10 Glyceryl Diisostearate, Emalex GWIS-210EX from Nihon Emulsion Company
- PEG-15 Glyceryl Diisostearate, Emalex GWIS-215
- PEG-20 Glyceryl Diisostearate, Emalex GWIS-220
- PEG-30 Glyceryl Diisostearate, Emalex GWIS-230
- PEG-60 Glyceryl Diisostearate, Emalex GWIS-260
- PEG-12 Glyceryl Dimyristate, Qusome-2 (BioZone Laboratories Inc.)
- PEG-4 Glyceryl Distearate, Emalex GWS-204
- PEG-12 Glyceryl Distearate, Qusome (BioZone Laboratories Inc.)
- PEG-23 Glyceryl Distearate, Inflacin (BioZone Laboratories Inc.)
- PEG-3 Glyceryl Triisostearate, Emalex GWIS-303 (Nihon Emulsion Company Ltd.)
- PEG-10 Glyceryl Triisostearate, Emalex GWIS-310
- PEG-15 Glyceryl Triisostearate, Emalex GWIS-315
- PEG-30 Glyceryl Triisostearate, Emalex GWIS-330
- PEG-40 Glyceryl Triisostearate, Emalex GWIS-340
- PEG-50 Glyceryl Triisostearate, Emalex GWIS-350
- PEG-60 Glyceryl Triisostearate, Emalex GWIS-360
- PEG-8 Glyceryl Trilaurate, M-Fineoil LCG (Miyoshi Oil & Fat Co. Ltd.)
- PEG-3 Glyceryl Trioleate, Emalex GWO-303
- PEG-5 Glyceryl Trioleate, Emalex GWO-305
- PEG-10 Glyceryl Trioleate, Emalex GWO-310
- PEG-15 Glyceryl Trioleate, Emalex GWO-315
- PEG-20 Glyceryl Trioleate, Emalex GWO-320
- PEG-25 Glyceryl Trioleate, Emalex GWO-325
- PEG-30 Glyceryl Trioleate, Emalex GWO-330
- PEG-40 Glyceryl Trioleate, Emalex GWO-340
- PEG-50 Glyceryl Trioleate, Emalex GWO-350
- PEG-60 Glyceryl Trioleate, Emalex GWO-360
- PEG-3 Glyceryl Tristearate, Emalex GWO-303
- PEG-4 Glyceryl Tristearate, Emalex GWO-304
- PEG-5 Glyceryl Tristearate, Emalex GWO-305
- PEG-6 Glyceryl Tristearate, Emalex GWO-306
- PEG-10 Glyceryl Tristearate, Emalex GWO-310
- PEG-15 Glyceryl Tristearate, Emalex GWO-315.

According to a preferred embodiment of the invention, the composition contains PEG-20 glyceryl triisostearate (INCI name: PEG-20 glyceryl triisostearate).

The nonionic surfactant can be present in a content ranging from 0.5% to 10% by weight, preferably ranging from 0.5% to 7% by weight and better still from 1% to 5% by weight, with respect to the total weight of the composition.

### Anhydrous medium

The composition according to the invention comprises an oily medium comprising at least one oil.

The term "oil" is understood to mean a fatty substance which is liquid at ambient temperature (25°C).

An oily medium which is suitable for the preparation of the cosmetic compositions according to the invention can comprise hydrocarbon oils, silicone oils, fluorinated or nonfluorinated oils, or their mixtures.

The oils can be volatile or nonvolatile.

They can be of animal, vegetable, mineral or synthetic origin. According to an alternative embodiment, oils of silicone origin are preferred.

Within the meaning of the present invention, the term "nonvolatile oil" is understood to mean an oil having a vapor pressure of less than 0.13 Pa.

Within the meaning of the present invention, the term "silicone oil" is understood to mean an oil comprising at least one silicon atom and in particular at least one Si-O group.

The term *"fluorinated oil"* is understood to mean an oil comprising at least one fluorine atom.

The term *"hydrocarbon oil"* is understood to mean an oil mainly containing hydrogen and carbon atoms.

The oils can optionally comprise oxygen, nitrogen, sulfur and/or phosphorus atoms, for example in the form of hydroxyl or acid radicals.

The term "*volatile oil*" is understood to mean, within the meaning of the invention, any oil which is capable of evaporating on contact with the skin in less than one hour, at ambient temperature and atmospheric pressure. The volatile oil is a volatile cosmetic compound which is liquid at ambient temperature and which especially has a nonzero vapor pressure, at ambient temperature and atmospheric pressure, which especially has a vapor pressure ranging from 0.13 Pa to 40 000 Pa (10⁻³ to 300 mmHg), in particular ranging from 1.3 Pa to 13 000 Pa (0.01 to 100 mmHg) and more particularly ranging from 1.3 Pa to 1300 Pa (0.01 to 10 mmHg).

### Volatile oils

The volatile oils can be hydrocarbon oils or silicone oils.

Mention may in particular be made, among the volatile hydrocarbon oils having from 8 to 16 carbon atoms, of branched C₈-C₁₆ alkanes, such as C₈-C₁₆ isoalkanes (also known as isoparaffins), isododecane, isodecane, isohexadecane and, for example, the oils sold under the trade names of Isopar or Permethyl, branched C₈-C₁₆ esters, such as isohexyl neopentanoate, and their mixtures. Preferably, the volatile hydrocarbon oil is chosen from volatile hydrocarbon oils having from 8 to 16 carbon atoms, and their mixtures, in particular from isododecane, isodecane or isohexadecane, and is in particular isohexadecane.

Mention may also be made of volatile linear alkanes comprising from 8 to 16 carbon atoms, in particular from 10 to 15 carbon atoms and more particularly from 11 to 13 carbon atoms, for example such as n-dodecane (C₁₂) and n-tetradecane (C₁₄), sold by Sasol under the respective references Parafol 12-97 and Parafol 14-97, and also their mixtures, the undecane/tridecane mixture, the mixtures of n-undecane (C₁₁) and of n-tridecane (C₁₃) obtained in Examples 1 and 2 of application WO 2008/155059 from Cognis, and their mixtures.

Mention may be made, as volatile silicone oils, of linear volatile silicone oils, such as hexamethyldisiloxane, octamethyltrisiloxane, decamethyltetrasiloxane, tetradecamethylhexasiloxane, hexadecamethylheptasiloxane and dodecamethylpentasiloxane.

Mention may be made, as volatile cyclic silicone oils, of hexamethylcyclotrisiloxane, octamethylcyclotetrasiloxane, decamethylcyclopentasiloxane and dodecamethylcyclohexasiloxane.

### Nonvolatile oils

The nonvolatile oils can be chosen in particular from nonvolatile hydrocarbon, fluorinated and/or silicone oils.

Mention may in particular be made, as nonvolatile hydrocarbon oil, of:
- hydrocarbon oils of animal origin,
- hydrocarbon oils of vegetable origin, synthetic ethers having from 10 to 40 carbon atoms, such as dicaprylyl ether,
- synthetic esters, such as oils of formula R₁COOR₂, in which Ri represents a residue of a linear or branched fatty acid comprising from 1 to 40 carbon atoms and R₂ represents a hydrocarbon chain, in particular a branched hydrocarbon chain, containing from 1 to 40 carbon atoms, provided that R₁ + R₂ is ≥ 10. The esters can be chosen in particular from esters of alcohol and of fatty acid, such as, for example, cetearyl octanoate, esters of isopropyl alcohol, such as isopropyl myristate or isopropyl palmitate, ethyl palmitate, 2-ethylhexyl palmitate, isopropyl stearate, octyl stearate, hydroxylated esters, such as isostearyl lactate or octyl hydroxystearate, alkyl or polyalkyl ricinoleates, hexyl laurate, esters of neopentanoic acid, such as isodecyl neopentanoate or isotridecyl neopentanoate, or esters of isononanoic acid, such as isononyl isononanoate or isotridecyl isononanoate,
- polyol esters and pentaerythritol esters, such as dipentaerythrityl tetrahydroxystearate/tetraisostearate,
- fatty alcohols which are liquid at ambient temperature comprising a branched and/or unsaturated carbon chain having from 12 to 26 carbon atoms, such as 2-octyldodecanol, isostearyl alcohol or oleyl alcohol,
- higher C₁₂-C₂₂ fatty acids, such as oleic acid, linoleic acid or linolenic acid, and their mixtures,
- nonphenylated silicone oils, such as, for example, caprylyl methicone, and
- phenylated silicone oils, such as, for example, phenyl trimethicones, phenyl dimethicones, phenyl(trimethylsiloxy)diphenylsiloxanes, diphenyl dimethicones, diphenyl(methyldiphenyl)trisiloxanes and (2-phenylethyl)trimethylsiloxysilicates, dimethicones or phenyl trimethicones with a viscosity of less than or equal to 100 cSt, trimethylpentaphenyltrisiloxane, and their mixtures; and also the mixtures of these different oils.

The amount of oil(s) can range, for example, from 30% to 94.3% by weight, preferably from 30% to 80% by weight, better still from 30% to 60% by weight and advantageously from 30% to 50% by weight, with respect to the total weight of the composition.

The composition can also comprise one or more solid fatty substances, such as waxes or pasty fatty substances.

These solid fatty substances can preferably be present in a proportion of 0.01% to 30% by weight and in particular of 0.1% to 20% by weight, with respect to the total weight of the composition.

The composition according to the invention can also comprise the ingredients normal in cosmetics, such as antioxidants, fragrances or preservatives other than C₃-C₁₀ diols.

The amounts of these different ingredients are those conventionally used in the fields under consideration, and for example from 0.01% to 20% of the total weight of the composition.

A person skilled in the art will take care to choose the ingredients participating in the composition, and also their amounts, so that they do not harm the properties of the compositions of the present invention.

According to a preferred embodiment, the composition according to the invention is a cosmetic composition intended to be applied to the skin and to be subsequently rinsed off, preferably with water.

The composition according to the invention can be provided in the form of a composition for exfoliating or scrubbing the skin of the body or of the face.

It can especially find a particularly advantageous application in the field of caring for and/or cleansing, in particular exfoliating, the skin of the body and/or of the face.

A cosmetic composition according to the invention can in particular be found in the form of a scrub for the face and/or for the body

According to the invention, a method for the cosmetic treatment of the skin of the body and/or of the face comprises a stage of application of a cosmetic composition according to the invention, preferably followed by a stage of rinsing off said composition, preferentially with water.

The amount of composition to be applied to the skin can be determined as a function of the area to be treated and of the desired intensity of the treatment.

During the application, the composition is advantageously massaged on the skin so that the exfoliating particles carry out an exfoliation (or scrubbing) of the skin by rubbing over the surface layers of the epidermis. The massaging of the skin can last from 1 minute to 10 minutes.

The application stage, generally comprising the massaging, can last from 1 minute to 1 hour, it being possible for the composition to be left in contact with the skin as in the case of a mask.

On conclusion of the application, the composition is generally rinsed off, typically with water, or else is wiped off.

Another subject matter of the invention is a process for the preparation of the composition described above, comprising a stage of mixing the oils, the C₃-C₁₀ diols, the surfactant and the additional thickeners, preferably by heating between 60 and 90°C, then a stage of addition of the silica aerogel and of the exfoliating particles, and finally a stage of cooling to ambient temperature.

The properties of the composition of the invention will now be illustrated in the following examples, which are nonlimiting of the scope of the invention.

### EXAMPLES

The following compositions were prepared according to the preparation process presented below.

A portion of the fatty substances is mixed with the preservatives and the mixture obtained is heated at 80°C in order to dissolve the preservatives. The remainder of the fatty substances and the fragrance are added and then, finally, the gelling agent(s), the surfactant and the colorants are added.

The mixture is stirred under a Rayneri stirrer throughout the time of the additions. Once the gel has been obtained, the sugars and then the alcohol are added

The viscosity of the product is evaluated at 25°C using the Rheomat (spindle 4, after 10 minutes of rotation).

The contents of each ingredient expressed by weight of active materials.

| Compositions | **Comparative Example 1** | **Comparative Example 2** | **Comparative Example 3** |
|---|---|---|---|
| Sucrose | **26** | **26** | **26** |
| Cane sugar (saccharose) | **12** | **12** | **12** |
| Shea butter | **4** | **4** | **4** |
| Isopropyl palmitate | **12** | **12** | **12** |
| Rapeseed oil | **15** | **15** | **15** |
| Coconut oil | **12.5** | **12.5** | **12.5** |
| Aerogel (3) | **3.5** | | |
| Aerosil 200 (6) | | **4.5** | |
| Aerosil R972 (7) | | | **4.5** |
| Polyoxyethylenated glyceryl triisostearate (4) | **2.5** | **2.5** | **2.5** |
| Pentane-1,2-diol | **3** | **3** | **3** |
| Propane-1,3-diol | **2** | **2** | **2** |
| Chlorphenesin | **0.3** | **0.3** | **0.3** |
| N-Octanoylglycine | **1** | **1** | **1** |
| Ethanol | **2** | **2** | **2** |
| Pentaerythrityl tetra(dibutylhydrox yhydrocinnamate) (5) | **0.07** | **0.07** | **0.07** |
| Fragrance | **3** | **3** | **3** |
| Colorants | q.s. | q.s. | q.s. |
| Dextrin palmitate (1) | | | |
| **MIXTURE OF MONO-, DI-AND TRIGLYCERIDES OF BEHENIC ACID** (2) | | | |
| Viscosity (poises) | 66 | 87 | 29 |
| Stability 3 weeks at 45°C | No, the particles separate out on settling | OK | No, release of oil at the surface |
| Cosmetic properties | Too fluid | More fluid Leaves a residue on the fingers after rinsing off with water. | More fluid |

| Compositions | **Example 4 In accordance** | **Example 5 In accordance** | **Example 6 In accordance** |
|---|---|---|---|
| Sucrose | **26** | **26** | **26** |
| Cane sugar (saccharose) | **12** | **12** | **12** |
| Shea butter | **4** | **4** | **4** |
| Isopropyl palmitate | **12** | **12** | **12** |
| Rapeseed oil | **15** | **15** | **15** |
| Coconut oil | **12.5** | **12.2** | **11.5** |
| Aerogel (3) | **4.5** | **4.5** | **4.5** |
| Aerosil 200 (6) | | | |
| Aerosil R972 (7) | | | |
| Polyoxyethylenated glyceryl triisostearate (4) | **2.5** | **2.5** | **2.5** |
| Pentanediol | **3** | **3** | **3** |
| Propanediol | **2** | **2** | **2** |
| Chlorphenesin | **0.3** | **0.3** | **0.3** |
| N-Octanoylglycine | **1** | **1** | **1** |
| Ethanol | **2** | **2** | **2** |
| Pentaerythrityl tetra(dibutylhydrox | **0.07** | **0.07** | **0.07** |
| yhydrocinnamate) (5) | | | |
| Fragrance | **3** | **3** | **3** |
| Colorants | q.s. | q.s. | q.s. |
| Dextrin palmitate (1) | | 0.3 | |
| **MIXTURE OF MONO-, DI-AND TRIGLYCERIDES OF BEHENIC ACID** (2) | | | 1 |
| Viscosity (poises) | 172 | 146 | 163 |
| Stability | OK | OK | OK |
| 3 weeks at 45°C | | | |
| Cosmetic properties | Good spreading Suppleness | idem | idem |
| | Good taking with the fingers | | |

| | | | |
|---|---|---|---|
| (1) Rheopearl TL2-OR from Chiba Flour Milling (2) Compritol 888 CG ATO from Gattefossé (3) Silica silylate (VM-2270, sold by Dow Corning) (4) Emalex GWIS-320EX, sold by Nihon Emulsion (5) Tinogard TT, sold by Ciba (6) Fumed silica (Aerosil 200 from Evonik Degussa) (7) Fumed silica | | | |

Only the compositions of Examples 4 to 6 according to the invention exhibit a good stability, and a good rheology which makes it possible to take the product with the fingers and good spreading over the skin.

## Claims

1. An anhydrous, in particular cosmetic, composition comprising, in an oily fatty phase, at least:
a) a C₃-C₁₀ diol,
b) hydrophobic silica aerogel particles in a solids content ranging from 3.7% to 6% by weight with respect to the total weight of the composition,
c) exfoliating particles in an amount ranging from 10% to 50% by weight with respect to the total weight of the composition,
said composition exhibiting, at a temperature of 25°C, a viscosity of between 10 and 20 Pa.s.

2. The composition as claimed in claim 1, in which the C₃-C₁₀ diol is chosen from propane-1,3-diol, pentane-1,2-diol, octane-1,2-diol, decane-1,2-diol and their mixtures, preferably from propane-1,3-diol, pentane-1,2-diol and their mixtures.

3. The composition as claimed in claim 1 or 2, comprising a C₃-C₁₀ diol in a content ranging from 1% to 10% by weight, preferably ranging from 2% to 8% by weight and preferentially ranging from 3% to 7% by weight, with respect to the total weight of the composition.

4. The composition as claimed in any one of the preceding claims, in which the hydrophobic silica aerogel particles exhibit a specific surface per unit of weight ranging from 500 to 1500 m²/g, preferably from 600 to 1200 m²/g and better still from 600 to 800 m²/g, and a size, expressed as volume-average diameter (D[0.5]), ranging from 1 to 1500 µm, better still from 1 to 1000 µm, preferably from 1 to 100 µm, in particular from 1 to 30 µm, more preferably from 5 to 25 µm, better still from 5 to 20 µm and even better still from 5 to 15 µm.

5. The composition as claimed in any one of the preceding claims, in which the hydrophobic silica aerogel particles are of silylated silica with the INCI name: silica silylate.

6. The composition as claimed in any one of the preceding claims, in which the hydrophobic silica aerogel particles are present in the composition in a solids content ranging from 4% to 5.5% by weight and preferably from 4% to 5% by weight, with respect to the total weight of the composition.

7. The composition as claimed in any one of the preceding claims, in which the exfoliating particles are chosen from particles of sugars, such as particles of saccharose, of sucrose, of fructose, of glucose, of galactose or of maltose, and preferably of saccharose or of sucrose.

8. The composition as claimed in any one of the preceding claims, in which the exfoliating particles are present in an amount ranging 25% to 45% by weight, with respect to the total weight of the composition.

9. The composition as claimed in any one of the preceding claims, comprising at least one nonionic surfactant chosen from polyoxyethylenated diesters or triesters of fatty acids and of glycerol having from 2 to 60 ethylene oxide units.

10. The composition as claimed in the preceding claim, in which the nonionic surfactant can be present in a content ranging from 0.5% to 10% by weight, preferably ranging from 0.5% to 7% by weight and better still from 1% to 5% by weight, with respect to the total weight of the composition.

11. The composition as claimed in any one of the preceding claims, comprising at least one other additional lipophilic thickener other than the hydrophobic silica aerogel particles, in particular chosen from esters of dextrin and of a fatty acid and (poly)esters of at least one fatty acid comprising from 20 to 24 carbon atoms and of glycerol.

12. The composition as claimed in the preceding claim, in which the dextrin ester is chosen from dextrin myristate and/or dextrin palmitate, and their mixtures, and more preferably dextrin palmitate, advantageously in a content of between 0.1% and 2% by weight, preferably between 0.1% and 1% by weight, with respect to the total weight of the composition.

13. The composition as claimed in claim 11, in which the (poly)ester is a mixture of mono-, di- and/or triesters of behenic acid and of glycerol, in a content of between 0.2% and 4% by total weight, preferably between 0.5% and 2% by weight, with respect to the total weight of the composition.

14. The composition as claimed in any one of the preceding claims, comprising from 30% to 94.3% by weight, preferably from 30% to 80% by weight, better still from 30% to 60% by weight and advantageously from 30% to 50% by weight of oil(s), with respect to the total weight of the composition.

15. A method for the cosmetic treatment of the skin of the body and/or of the face, comprising a stage of application of a cosmetic composition as claimed in one of the preceding claims to the skin, preferably followed by a stage of rinsing off said composition, preferentially with water.

## Patentansprüche

1. Wasserfreie, insbesondere kosmetische, Zusammensetzung, umfassend, in einer öligen Fettphase, mindestens:
a) ein C₃-C₁₀-Diol,
b) hydrophobe Silica-Aerogel-Partikel in einem Feststoffgehalt im Bereich von 3,7 bis 6 Gew.-% bezogen auf das Gesamtgewicht der Zusammensetzung,
c) Peelingpartikel in einer Menge im Bereich von 10 bis 50 Gew.-% bezogen auf das Gesamtgewicht der Zusammensetzung,
wobei die Zusammensetzung bei einer Temperatur von 25°C eine Viskosität zwischen 10 und 20 Pa.s aufweist.

2. Zusammensetzung nach Anspruch 1, bei der das C₃-C₁₀-Diol ausgewählt ist aus Propan-1,3-diol, Pentan-1,2-diol, Octan-1,2-diol, Decan-1,2-diol und deren Mischungen, vorzugsweise aus Propan-1,3-diol, Pentan-1,2-diol und deren Mischungen.

3. Zusammensetzung nach Anspruch 1 oder 2, umfassend ein C₃-C₁₀-Diol in einem Gehalt im Bereich von 1 bis 10 Gew.-%, vorzugsweise im Bereich von 2 bis 8 Gew.-% und bevorzugt im Bereich von 3 bis 7 Gew.-%, bezogen auf das Gesamtgewicht der Zusammensetzung.

4. Zusammensetzung nach einem der vorhergehenden Ansprüche, bei der die hydrophoben Silica-Aerogel-Partikel eine spezifische Oberfläche pro Gewichtseinheit im Bereich von 500 bis 1500 m²/g, vorzugsweise 600 bis 1200 m²/g und besser noch 600 bis 800 m²/g, und eine Größe, ausgedrückt als volumengemittelter Durchmesser (D[0,5]), im Bereich von 1 bis 1500 µm, besser noch 1 bis 1000 µm, vorzugsweise 1 bis 100 µm, insbesondere 1 bis 30 µm, weiter bevorzugt 5 bis 25 µm, besser noch 5 bis 20 µm und noch besser 5 bis 15 µm, aufweisen.

5. Zusammensetzung nach einem der vorangehenden Ansprüche, bei der die hydrophoben Silica-Aerogel-Partikel bestehen aus silylierter Kieselsäure mit dem INCI-Namen: silica silylate.

6. Zusammensetzung nach einem der vorhergehenden Ansprüche, bei der die hydrophoben Silica-Aerogel-Partikel in der Zusammensetzung in einem Feststoffgehalt im Bereich von 4 bis 5,5 Gew.-% und vorzugsweise im Bereich von 4 bis 5 Gew.-%, bezogen auf das Gesamtgewicht der Zusammensetzung, vorhanden sind.

7. Zusammensetzung nach einem der vorhergehenden Ansprüche, bei der die Peelingpartikel aus Zuckerpartikeln, wie Saccharose-, Sucrose-, Fructose-, Glucose-, Galactose- oder Maltosepartikeln, und vorzugsweise aus Saccharose- oder Sucrosepartikeln, ausgewählt sind.

8. Zusammensetzung nach einem der vorhergehenden Ansprüche, bei der die Peelingpartikel in einer Menge im Bereich von 25 bis 45 Gew.-%, bezogen auf das Gesamtgewicht der Zusammensetzung, vorhanden sind.

9. Zusammensetzung nach einem der vorangegangenen Ansprüche, umfassend mindestens ein nichtionisches Tensid, ausgewählt aus den polyoxyethylenierten Diestern oder Triestern von Fettsäuren und Glycerin mit 2 bis 60 Ethylenoxideinheiten.

10. Zusammensetzung nach dem vorhergehenden Anspruch, bei der das nichtionische Tensid in einem Gehalt im Bereich von 0,5 bis 10 Gew.-%, vorzugsweise im Bereich von 0,5 bis 7 Gew.-% und noch besser im Bereich von 1 bis 5 Gew.-%, bezogen das Gesamtgewicht der Zusammensetzung, vorhanden sein kann.

11. Zusammensetzung nach einem der vorhergehenden Ansprüche, die außer den hydrophoben Silica-Aerogel-Partikeln mindestens ein weiteres zusätzliches lipophiles Verdickungsmittel enthält, das insbesondere unter den Estern von Dextrin und einer Fettsäure und den (Poly)estern von mindestens einer Fettsäure mit 20 bis 24 Kohlenstoffatomen und Glycerin ausgewählt ist.

12. Zusammensetzung nach dem vorhergehenden Anspruch, bei der der Dextrinester ausgewählt ist aus Dextrinmyristat und/oder Dextrinpalmitat und deren Mischungen, und noch bevorzugter aus Dextrinpalmitat, vorteilhafterweise in einem Gehalt zwischen 0,1 und 2 Gew.-%, vorzugsweise zwischen 0,1 und 1 Gew.-%, bezogen auf das Gesamtgewicht der Zusammensetzung.

13. Zusammensetzung nach Anspruch 11, bei der der (Poly)ester eine Mischung aus Mono-, Di- und/oder Triestern der Behensäure und Glycerin in einem Gehalt zwischen 0,2 und 4 Gew.-%, vorzugsweise zwischen 0,5 und 2 Gew.-%, bezogen auf das Gesamtgewicht der Zusammensetzung, ist.

14. Zusammensetzung nach einem der vorhergehenden Ansprüche, umfassend 30 bis 94,3 Gew.-%, vorzugsweise 30 bis 80 Gew.-%, besser noch 30 bis 60 Gew.-% und vorteilhafterweise 30 bis 50 Gew.-% Öl(e), bezogen auf das Gesamtgewicht der Zusammensetzung.

15. Verfahren zur kosmetischen Behandlung der Haut des Körpers und/oder des Gesichts, umfassend eine Stufe des Auftragens einer kosmetischen Zusammensetzung nach einem der folgenden Ansprüche auf die Haut, vorzugsweise gefolgt von einer Stufe des Abspülens der Zusammensetzung, vorzugsweise mit Wasser.

## Revendications

1. Composition anhydre, en particulier cosmétique, comprenant, dans une phase grasse huileuse, au moins :
a) un diol en C₃ à C₁₀,
b) des particules d'aérogel de silice hydrophobe en une teneur en solides située dans la plage allant de 3,7 % à 6 % en poids par rapport au poids total de la composition,
c) des particules exfoliantes en une quantité située dans la plage allant de 10 % à 50 % en poids par rapport au poids total de la composition,
ladite composition présentant, à une température de 25°C, une viscosité comprise entre 10 et 20 Pa.s.

2. Composition selon la revendication 1, dans laquelle le diol en C₃ à C₁₀ est choisi parmi le propane-1,3-diol, le pentane-1,2-diol, l'octane-1,2-diol, le décane-1,2-diol, et leurs mélanges, de préférence parmi le propane-1,3-diol, le pentane-1,2-diol, et leurs mélanges.

3. Composition selon la revendication 1 ou 2, comprenant un diol en C₃ à C₁₀ en une teneur située dans la plage allant de 1 % à 10 % en poids, de préférence de 2 % à 8 % en poids, et préférentiellement de 3 % à 7 % en poids, par rapport au poids total de la composition.

4. Composition selon l'une quelconque des revendications précédentes, dans laquelle les particules d'aérogel de silice hydrophobe présentent une surface spécifique par unité de masse située dans la plage allant de 500 à 1500 m²/g, de préférence de 600 à 1200 m²/g et mieux de 600 à 800 m²/g, et une taille, exprimée en diamètre moyen en volume (D[0,5]), située dans la plage allant de 1 à 1500 µm, mieux de 1 à 1000 µm, de préférence de 1 à 100 µm, en particulier de 1 à 30 µm, plus préférablement encore de 5 à 25 µm, mieux de 5 à 20 µm et encore plus préférablement de 5 à 15 µm.

5. Composition selon l'une quelconque des revendications précédentes, dans laquelle les particules d'aérogel de silice hydrophobe sont constituées de silice silylée dont le nom INCI est le silylate de silice.

6. Composition selon l'une quelconque des revendications précédentes, dans laquelle les particules d'aérogel de silice hydrophobe sont présentes dans la composition en une teneur en solides située dans la plage allant de 4 % à 5,5 % en poids et de préférence de 4 % à 5 % en poids par rapport au poids total de la composition.

7. Composition selon l'une quelconque des revendications précédentes, dans laquelle les particules exfoliantes sont choisies parmi des particules de sucres, telles que des particules de saccharose, de sucrose, de fructose, de glucose, de galactose, ou de maltose, de préférence de saccharose ou de sucrose.

8. Composition selon l'une quelconque des revendications précédentes, dans laquelle les particules exfoliantes sont présentes en une quantité située dans la plage allant de 25 % à 45 % en poids par rapport au poids total de la composition.

9. Composition selon l'une quelconque des revendications précédentes, comprenant au moins un tensioactif non ionique choisi parmi les diesters ou triesters polyoxyéthylénés d'acides gras et de glycérol ayant de 2 à 60 motifs d'oxyde d'éthylène.

10. Composition selon la revendication précédente, dans laquelle le tensioactif non ionique peut être présent en une teneur située dans la plage allant de 0,5 % à 10 % en poids, de préférence de 0,5 % à 7 % en poids et encore mieux de 1 % à 5 % en poids par rapport au poids total de la composition.

11. Composition selon l'une quelconque des revendications précédentes, comprenant au moins un autre épaississant lipophile additionnel différent des particules d'aérogels de silice hydrophobe, en particulier choisi parmi les esters de dextrine et d'acide gras, et les (poly)esters d'au moins un acide gras comprenant de 20 à 24 atomes de carbone et de glycérol.

12. Composition selon la revendication précédente, dans laquelle l'ester de dextrine est choisi parmi le myristate de dextrine et/ou le palmitate de dextrine, et leurs mélanges, et plus préférentiellement le palmitate de dextrine, avantageusement en une teneur comprise entre 0,1 % et 2 % en poids, de préférence entre 0,1 % et 1 % en poids par rapport au poids total de la composition.

13. Composition selon la revendication 11, dans laquelle le (poly)ester est un mélange de mono-, di- et/ou tri-esters d'acide béhénique et de glycérol, en une teneur comprise entre 0,2 % et 4 % en poids total, de préférence entre 0,5 % et 2 % en poids par rapport au poids total de la composition.

14. Composition selon l'une quelconque des revendications précédentes, comprenant de 30 % à 94,3 % en poids, de préférence de 30 % à 80 % en poids, mieux de 30 % à 60 % et avantageusement de 30 % à 50 % en poids d'huile(s) par rapport au poids total de la composition

15. Procédé de traitement cosmétique de la peau du corps et/ou du visage, comprenant une étape d'application d'une composition cosmétique selon l'une des revendications précédentes sur la peau, de préférence suivie d'une étape de rinçage, préférentiellement à l'eau, de ladite composition.
